# EUROPÄISCHE PATENTANMELDUNG

(11) **EP 1 839 700 A2**
(43) Veröffentlichungstag der Anmeldung: **03.10.2007**
(21) Anmeldenummer: 07004106.6
(22) Anmeldetag: 28.02.2007
(51) Int. Cl.: A61N 1/05

(54) **Medizinische, implantierbare Elektrodenvorrichtung**

(30) Priorität: 30.03.2006 DE 102006014698
(71) Anmelder: BIOTRONIK CRM Patent AG, 6341 Baar (CH)
(72) Erfinder: Kolberg, Gernot, 12043 Berlin (DE)
(74) Vertreter: Lindner-Vogt, Karin L.

(57) **Zusammenfassung**

Eine medizinische, implantierbare Elektrodenvorrichtung, insbesondere kardiologische Elektrodenvorrichtung, umfasst einen langgestreckten Elektrodenkörper (2) mit einem proximalen und einem distalen Ende (1) zum Einführen in den Körper eines Patienten sowie mehrere, zur Fixierung der Elektrodenvorrichtung im Patienten vor dem distalen Ende (1) seitlich am Elektrodenkörper (2) angebrachte, über dessen Umfang verteilte, strebenartige Verankerungselemente (4), die jeweils mit ihrer Längsachse (SL) in einem spitzen, sich in Richtung proximales Ende öffnenden Winkel (W1) bezogen auf die Elektrodenkörperachse (KL) abstehen. Die Verankerungselemente besitzen in Umfangsrichtung eine Vorzugsrichtung derart besitzen, dass sie bei Eingriff der Verankerungselemente (4) in eine Körperpartie des Patienten einer Drehung des Elektrodenkörpers (2) in einer Drehrichtung einen größeren Widerstand entgegensetzen als in die Gegenrichtung.

## Beschreibung

Die Erfindung betrifft eine medizinische, implantierbare Elektrodenvorrichtung und insbesondere eine kardiologische Elektrodenvorrichtung wie beispielsweise einen Herzkatheter mit den im Oberbegriff des Anspruches 1 angegebenen Merkmalen.

Auf dem Gebiet von Herzelektroden ist es üblich, im Bereich des distalen Endes des langgestreckten Elektrodenkörpers, das in den Patientenkörper eingeführt wird, seitlich am Elektrodenkörper über dessen Umfang verteilt Verankerungselemente anzubringen, die zur Fixierung der Elektrodenvorrichtung im Körper des Patienten dienen. Diese Verankerungselemente sind beispielsweise aus weichem Kunststoff an den Elektrodenkörper angespritzte, flügelartige Streben, die sich im Trabekelwerk des Ventrikels verhaken und so für eine Fixierung des Elektrodenkörpers sorgen sollen. Diese gemeinhin als "Tines"' bezeichneten Verankerungselemente stehen dabei mit ihrer Längsachse jeweils in einem spitzen, sich in Richtung des proximalen Endes des Elektrodenkörpers öffnenden Winkels bezogen auf die Elektrodenkörperachse davon ab, um einem Herausziehen der Elektrodenspitze aus ihrem Verankerungspunkt entgegenzuwirken.

Weitere Anwendungsfälle für die vorliegende Erfindung können auch in Gefäße implantierbare Elektroden, wie beispielsweise eine Koronarsinuselektrode sein, die ihrerseits über Verankerungselemente entlang ihres Elektrodenkörpers - also durchaus mit einigem proximalen Abstand vor dem distalen Ende - im Koronarsinus festgelegt ist.

Bei den bekannten Elektrodenvorrichtungen kann das Problem auftreten, dass sich die Verankerungselemente beim Einsetzen oder Herausziehen des Katheters an anderen Körperstrukturen, wie beispielsweise in den Klappenfäden, verheddern können. Dies führt dazu, dass die Elektrode mit verhältnismäßig starkem Zug am Elektrodenkörper befreit werden muss, was zu lokalen Beeinträchtigungen des Herzgewebes führen kann.

Lösungsansätze für diese Problematik der Verankerungselemente wurden dahingehend vorgeschlagen, dass diese durch eine mechanische Steuerung ein- und ausgefahren werden können. Dies ist allerdings konstruktiv sehr aufwändig und aufgrund der sehr filigranen Strukturen der Verankerungselemente auch anfällig.

Davon ausgehend liegt der Erfindung die Aufgabe zugrunde, die bekannten Elektrodenvorrichtungen hinsichtlich ihrer Verankerungselemente so zu verbessern, dass ein störungsärmeres Im- und Explantieren der Elektrodenvorrichtung, dabei jedoch deren zuverlässige Fixierung gewährleistet werden.

Diese Aufgabe wird durch die im Kennzeichnungsteil des Anspruches 1 angegebenen Merkmale gelöst. Demnach besitzen die Verankerungselemente in Umfangsrichtung eine Vorzugsrichtung in der Art, dass sie bei Eingriff der Verankerungselemente in eine Körperpartie des Patienten einer Drehung des Elektrodenkörpers in einer vorgegebenen Drehrichtung einen größeren Widerstand entgegensetzen als in die Gegenrichtung.

Diese Auslegung führt dazu, dass der Implanteur bei einem Verheddern der Verankerungselemente beispielsweise in den Klappenfäden den Elektrodenkörper in diejenige Drehrichtung in Rotation versetzt, in der er einen geringeren Drehwiderstand spürt. Diese Rotationsanisotropie kann beispielsweise durch eine verkippte Anordnung der Verankerungselemente bezogen auf eine von der Elektrodenkörperachse und dem Fußpunkt des jeweiligen Verankerungselementes aufgespannten Radialebene erzielt werden. Die strebenartigen Verankerungselemente stehen also bezogen auf die Umfangsrichtung schräg vom Elektrodenkörper ab. Dies bedeutet, dass sie sich bei Drehung in einer Richtung bei einem äußeren Widerstand, wie beispielsweise durch das Trabekelwerk im Herzen selbst, aufstellen und damit sich stärker mit dem umliegenden Gewebe verkrallen. Bei einer Drehung in Gegenrichtung schmiegen sich die Verankerungselemente an den Elektrodenkörper an, sodass sie außer Eingriff mit dem äußeren Gewebe gelangen und somit ein einfacheres Herausziehen des Elektrodenkörpers aus seiner Fixierung beispielsweise im Trabekelwerk oder ein leichteres Lösen aus einer Verhedderung in den Klappenfäden erzielbar sind.

Gemäß bevorzugten Ausführungsformen kann die angesprochene Richtungsanisotropie der Verankerungselemente auch durch eine in Umfangsrichtung bezogen auf den Elektrodenkörper bogenförmige Ausbildung oder durch eine entsprechende anisotrope Biegesteifigkeit der Verankerungselemente erzielt werden.

Die Erfindung kann auch bei einer Gefäßelektrode, wie z. B. einer Koronarsinuselektrode angewendet werden. Bei einer entsprechenden Weiterbildung sind die Verankerungselemente durch mehrere, über den Umfang des Elektrodenkörpers verteilte, im Wesentlichen radial abstehende und sich in Längsrichtung des Elektrodenkörpers erstreckende Lamellen gebildet, die bezogen auf die Radialrichtung einen gebogenen Verlauf aufweisen.

Letzterer setzt ebenfalls das Grundkonzept der Erfindung um, nämlich der bezogen auf die Drehung des Elektrodenkörpers anisotropen Widerstand. So kann beispielsweise durch eine Rechtsdrehung des Elektrodenkörpers ein Aufspreizen der abstehenden Lamellen erzielt werden, was eine sichere Verankerung der Elektrode in einem Gefäß hervorruft. Weitere Merkmale, Vorteile und Einzelheiten der Erfindung sind der nachfolgenden Beschreibung entnehmbar, in der Ausführungsbeispiele anhand der beigefügten Zeichnungen näher erläutert werden. Es zeigen:
- Fig. 1: eine Seitenansicht des distalen Endes eines Herzkatheters,
- Fig. 2: eine Ansicht des distalen Endes aus Pfeilrichtung II gemäß Fig. 1,
- Fig. 3: eine Frontansicht analog Fig. 2 eines Herzkatheters in einer alternativen Ausführungsform,
- Fig. 4: eine ausschnittsweise perspektivische Ansicht einer Verankerungszone einer Koronarsinuselektrode, und
- Fig. 5: eine schematische Darstellung der Elektrode gemäß Fig. 4 in ihrer Position im Koronarsinus.

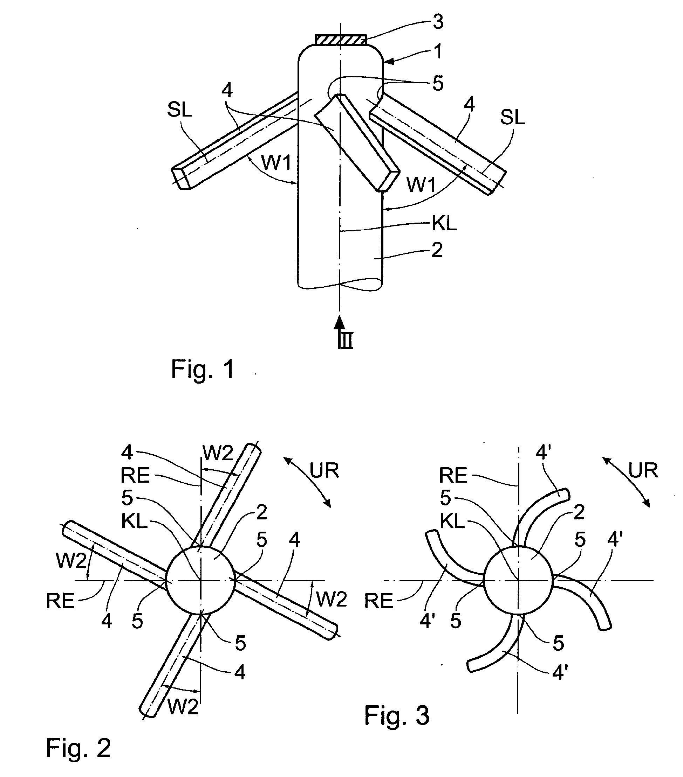

Die beiden Zeichnungen gemäß Fig. 1 und 2 zeigen das distale Ende 1 eines Elektrodenkörpers 2 eines Herzkatheters, das beispielsweise im Trabekelwerk im Ventrikel des Herzens zu verankern ist. Dort kann über die in Fig. 1 gezeigte Spitzenelektrode 3 ein kardiologisches Potential gemessen und/oder ein Spannungsimpuls beispielsweise bei Anwendung des Herzkatheters mit einem Herzschrittmacher abgegeben werden.

Zur Verankerung des distalen Endes 1 mit seiner Spitzenelektrode 3 sind am Elektrodenkörper 2 vor dem Ende seitlich abstehende, über den Umfang verteilte Verankerungsstreben aus einem elastischen Kunststoffmaterial - z.B. Silikon - direkt an den Elektrodenkörper 2 angespritzt. Diese Verankerungsstreben 4 stehen einerseits mit ihrer Längsachse SL in einem spitzen, sich in Richtung proximales Ende (nicht dargestellt) öffnenden Winkel W1 von beispielsweise 60 ° zur Körperlängsachse KL des Elektrodenkörpers 2. Wie aus Fig. 1 und deutlicher aus Fig. 2 hervorgeht, sind die vier Verankerungsstreben 4 außerdem jeweils aus der von der Körperlängsachse KL und dem jeweiligen Fußpunkt 5 der Verankerungsstrebe 4 aufgespannten Radialebene RE um einen spitzen Winkel W2 herausgekippt. Der Winkel W2 liegt vorzugsweise bei etwa 30 °, wobei die Kipprichtung einer jeden Verankerungsstrebe 4 jeweils - bezogen auf eine Betrachtungsrichtung ausgehend vom proximalen Ende 1 - im Uhrzeigersinn weist. Dies hat zur Folge, dass bei einer Drehung des Elektrodenkörpers 2 im Uhrzeigersinn beispielsweise zum Verankern des distalen Endes 1 im Trabekelwerk des Ventrikels sich zum einen ein verstärkter Verankerungseffekt, zum anderen für den Implanteur eine fühlbare Drehrichtungsabhängigkeit des Widerstandes einstellen. Beim "Einschrauben" des Elektrodenkörpers 2, also bei einer Drehung bezogen auf Fig. 2 im Uhrzeigersinn, stellen sich die Verankerungsstreben 4 gegen das Trabekelwerk auf, was zu einer Vergrößerung des Drehwiderstandes führt. Der Implanteur spürt also intuitiv, dass sich das distale Ende 1 im Trabekelwerk verankert.

Zum Lösen des Elektrodenkörpers 2 aus dem Trabekelwerk - oder auch beim Störfall eines Verhedderns der Verankerungsstreben 4 etwa in den Klappenfäden - wird der Elektrodenkörper 2 entgegen dem Uhrzeigersinn gedreht, also "herausgeschraubt", wobei sich die Verankerungsstreben 4 erkennbar an den Elektrodenkörper 2 anlegen und der Eingriff der Verankerungsstreben 4 in das Trabekelwerk damit merklich reduziert wird. Ein weiterer Vorteil der Verkippung der Verankerungsstreben 4 liegt darin, dass sich bei einem "Einschrauben" die Verankerungsstreben 4 hinter Vorsprünge im Körper oder unter Hinterschneidungsbereiche schieben können, die ohne die Kippstellung nicht erreichbar wären. Auch kann sich der Implanteur durch den sich bildenden Drehwiderstand einen Eindruck vom Zustand der Fixierung der Elektrode an der entsprechenden Stelle am Herzen verschaffen. Er bekommt also ein "Gefühl" für den Fixiergrad, ohne an der Elektrode ziehen zu müssen.

In Fig. 3 ist eine Alternative für die Erzeugung eines anisotropen Drehwiderstandes dargestellt. Dort setzen die Verankerungsstreben 4' zwar koplanar mit der Radialebene RE an den Elektrodenkörper 2 an, sind aber im Uhrzeigersinn bogenförmig gestaltet, wodurch sich auch hier beim Drehen im Uhrzeigersinn bezogen auf Fig. 3 eine Widerstandverstärkung, beim Drehen entgegen dem Uhrzeigersinn eine Abschwächung des Widerstandes ergeben. Die Verankerungsstreben 4' stehen im Übrigen analog den Streben 4 gemäß Fig. 1 und 2 in einem spitzen Winkel W1 zur Körperlängsachse KL.

Wie zeichnerisch nicht näher dargestellt ist, kann sich die Drehrichtung-Anisotropie auch durch eine anisotrope Biegesteifigkeit der jeweiligen Verankerungsstrebe 4 bezogen auf die Umfangsrichtung UR einstellen.

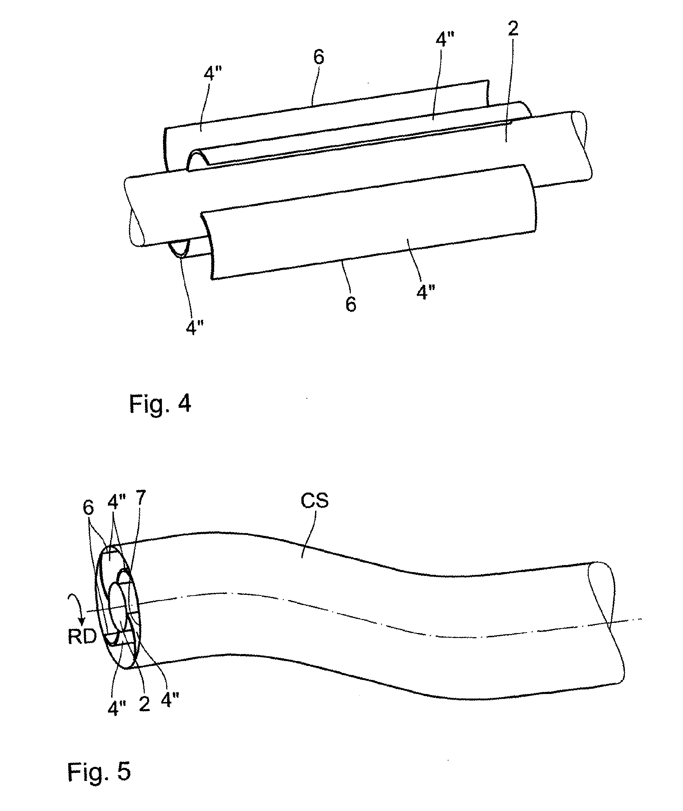

Die in Fig. 4 und 5 gezeigte Ausführungsform der Elektrodenvorrichtung gibt (ausschnittsweise) eine Koronarsinuselektrode wieder, deren Elektrodenkörper 2 gegebenenfalls an mehreren Positionen proximal vor ihrem distalen Ende (hier nicht dargestellt) mit Verankerungselementen in Form von Lamellen 4" versehen ist. Vier dieser Lamellen 4" sind über den Umfang des Elektrodenkörpers 2 gleichmäßig und erstrecken sich mit ihrer Hauptrichtung in Längsrichtung des Elektrodenkörpers 2. Bezogen auf die Radialrichtung weisen die Lamellen 4" einen gebogenen Verlauf auf, wie dies aus den Fig. 4 und 5 deutlich wird.

Der Zweck des gebogenen Verlaufes wird aus Fig. 5 deutlich. Die Lamellen 4" spreizen sich bei einer Rechtsdrehung RD des Elektrodenkörpers 2 durch die voreilende Stirnkante 6 der Lamellen 4" gegen die Gefäßwand 7 auf, wodurch eine gute Verankerung der Elektrode im Koronarsinus CS erzielbar ist

## Patentansprüche

1. Medizinische, implantierbare Elektrodenvorrichtung, insbesondere kardiologische Elektrodenvorrichtung, umfassend
- einen langgestreckten Elektrodenkörper (2) mit einem proximalen und einem distalen Ende (1) zum Einführen in den Körper eines Patienten, und
- mehrere, zur Fixierung der Elektrodenvorrichtung im Patienten proximal vor dem distalen Ende (1) seitlich am Elektrodenkörper (2) angebrachte, über dessen Umfang verteilte Verankerungselemente (4, 4', 4"),
**dadurch gekennzeichnet, dass**
- die Verankerungselemente (4, 4', 4") in Umfangsrichtung (UR) eine Vorzugsrichtung derart besitzen, dass sie bei Eingriff der Verankerungselemente (4, 4', 4") in eine Körperpartie des Patienten einer Drehung des Elektrodenkörpers (2) in einer Drehrichtung einen größeren Widerstand entgegensetzen als in die Gegenrichtung.

2. Elektrodenvorrichtung nach Anspruch 1, **dadurch gekennzeichnet, dass** der größere Drehwiderstand bei Drehung des Elektrodenkörpers (2) im Uhrzeigersinn vom proximalen Ende aus gesehen auftritt.

3. Elektrodenvorrichtung nach Anspruch 1 oder 2 mit strebenartigen Verankerungselementen (4, 4'), die jeweils mit ihrer Längsachse (SL) in einem spitzen, sich in Richtung proximales Ende öffnenden Winkel (W1) bezogen auf die Elektrodenkörperachse (KL) abstehen, **dadurch gekennzeichnet, dass** die Verankerungselemente (4) aus einer von der Elektrodenkörperachse (KL) und dem Fußpunkt (5) des jeweiligen Verankerungselementes (4, 4') aufgespannten Radialebene (RE) um einen spitzen Winkel (W2) herausgekippt angeordnet sind.

4. Elektrodenvorrichtung nach Anspruch 3, **dadurch gekennzeichnet, dass** der Kippwinkel (W2) zwischen 5° und 45°, vorzugsweise bei 30°, liegt.

5. Elektrodenvorrichtung nach einem der vorgenannten Ansprüche, **dadurch gekennzeichnet, dass** die Verankerungselemente (4') in Umfangsrichtung (UR) bezogen auf den Elektrodenkörper (2) bogenförmig ausgebildet sind.

6. Elektrodenvorrichtung nach Anspruch 1 oder 2, **dadurch gekennzeichnet, dass** die Verankerungselemente durch mehrere, über den Umfang des Elektrodenkörpers (2) verteilte, im Wesentlichen radial abstehende und sich in Längsrichtung des Elektrodenkörpers (2) erstreckende Lamellen (4") gebildet sind, die bezogen auf die Radialrichtung einen gebogenen Verlauf aufweisen.

7. Elektrodenvorrichtung nach Anspruch 6, **dadurch gekennzeichnet, dass** drei bis sechs, vorzugsweise vier Lamellen (4") vorgesehen sind.

8. Elektrodenvorrichtung nach einem der vorgenannten Ansprüche, **dadurch gekennzeichnet, dass** die Biegesteifigkeit des jeweiligen Verankerungselementes in Umfangsrichtung (UR) anisotrop ist.
